# EUROPEAN PATENT APPLICATION

(11) **EP 3 106 514 A1**
(43) Date of publication of application: **21.12.2016**
(21) Application number: 14881916.2
(22) Date of filing: 10.02.2014
(51) Int. Cl.: C12N 1/14, A01N 63/04

(54) **METHOD FOR PRODUCING CELL EXTRACT HAVING AGRICULTURAL PEST INSECTICIDAL CAPACITY AND METHOD FOR INSECTICIDE OF AGRICULTURAL PEST**

(71) Applicant: Towada Green Tuff Agro-Science Co., Ltd., Tokyo 152-0011 (JP); Kinki University, Higashiosaka-shi, Osaka 577-0818 (JP)
(72) Inventor: ANO Takashi, Kinokawa-shi Wakayama 649-6493 (JP); HIROSE Yoichiro, Tokyo 105-0012 (JP)
(74) Representative: Novagraaf Technologies
(86) International application number: PCT/JP2014/053082
(87) International publication number: WO 2015/118689

(57) **Abstract**

Provided are a method for producing a cell extract having agricultural pest insecticidal capacity by a novel method for culturing filamentous fungi having insecticidal capacity for an agricultural pest, and a method for insecticide having an exceptional immediate effect within a short period of time on an agricultural pest due to filamentous fungi contained in a cell extract produced by this production method. A method for producing a cell extract having agricultural pest insecticidal capacity provided with a step for culturing filamentous fungi in solid medium and obtaining a solid culture of the filamentous fungi, a step for culturing the filamentous fungi in liquid medium and obtaining a liquid culture of the filamentous fungi, a step for separating mycelia of the filamentous fungi from the liquid culture of the filamentous fungi, a step for mixing the liquid culture from which the mycelia have been removed and the solid culture and obtaining a mixed culture, and a step for extracting a spore solution of the filamentous fungi from this mixed culture; and a method for insecticide of an agricultural pest that causes a cell extract obtained by this production method to act on an agricultural pest or a habitat thereof.

## Description

### Technical Field

The present invention relates to a method for producing a cell extract of filamentous fungi having agricultural pest insecticidal capacity and a method for insecticide of an agricultural pest.

### Background Art

A chemical pesticide has an immediate effect on an agricultural pest, but safety concerns with respect to a non-target are known. Meanwhile, various proposals have been made for a microbial pesticide having agricultural pest insecticidal capacity, which has been said to be better in safety. However, any one of these is a slow-acting insecticide which lacks an immediate effect to perform insecticide by causing infection slowly.

Various microorganisms used for the microbial pesticide are known. Among these microorganisms, a study that filamentous fungi Trichoderma album classified into the genus Trichoderma lives on Dermanyssus gallinae to kill Dermanyssus gallinae has been published (Non-Patent Literature 1).

In bacteria belonging to the genus Trichoderma, Trichoderma atroviride distributed widely in the world is a microorganism always existing widely in a natural environment such as soil or a bacteria floor. When a microbial pesticide is produced using this microorganism, a method for culturing a large amount of Trichoderma atroviride identified from an isolated source such as soil in an environment different from an isolated location using a large tank or an industrial culture facility, and then causing the culture to be supported on silica or other mineral fine powder is known.

### Citation List

### Patent Literature

Patent Literature 1: JP 2008-61530 A

### Non-Patent Literature

Non-Patent Literature 1: Hussein A. Kaoud et al, susceptibility of Poultry Red mites to Entomopathogens", International Journal of Poultry Science 9 (3), p. 259-263
Non-Patent Literature 2: "EFSA assesses potential link between two neonicotinoids and developmental neurotoxicity", EFSA Press Release, December 17, 2013

### Summary of Invention

### Technical Problem

An object of the present invention is to provide a method for producing a cell extract having agricultural pest insecticidal capacity by a novel method for culturing filamentous fungi having agricultural pest insecticidal capacity, and an effective method for insecticide having an exceptional immediate effect within a short period of time on an agricultural pest due to filamentous fungi contained in a cell extract produced by the production method.

### Solution to Problem

In order to solve the above problem, the following inventions will be proposed.

An invention according to claim 1 is a method for producing a cell extract having agricultural pest insecticidal capacity, including the following steps.
(1) Step for culturing filamentous fungi in a solid medium and obtaining a solid culture of the filamentous fungi
(2) Step for culturing the filamentous fungi in a liquid medium and obtaining a liquid culture of the filamentous fungi
(3) Step for separating mycelia of the filamentous fungi from the liquid culture of the filamentous fungi
(4) Step for mixing the liquid culture from which the mycelia have been removed and the solid culture and obtaining a mixed culture
(5) Step for extracting a spore solution of the filamentous fungi from the mixed culture

An invention according to claim 2 is the method for producing a cell extract according to claim 1, further including a step for mixing the spore solution and an inorganic mineral powder aqueous solution.

An invention according to claim 3 is the method for producing a cell extract according to claim 1 or 2, in which the filamentous fungi belong to the genus Trichoderma.

An invention according to claim 4 is a method for insecticide of an agricultural pest, in which a cell extract obtained by the production method according to any one of claims 1 to 3 is acted on an agricultural pest or a habitat thereof.

An invention according to claim 5 is a method for insecticide of an agricultural pest, in which a mixed solution obtained by adding vegetable oil to a supernatant of a cell extract obtained by the production method according to any one of claims 1 to 3 is acted on an agricultural pest or a habitat thereof.

An invention according to claim 6 is a method for insecticide of an agricultural pest, in which a mixed solution obtained by adding vegetable oil to a supernatant of a cell extract obtained by the production method according to any one of claims 1 to 3 is diluted, and the diluted mixed solution is acted on an agricultural pest or a habitat thereof.

An invention according to claim 7 is the method for insecticide according to any one of claims 4 to 6, in which the filamentous fungi belong to the genus Trichoderma.

### Advantageous Effects of Invention

The present invention can provide a method for producing a cell extract having agricultural pest insecticidal capacity by a novel method for culturing filamentous fungi having agricultural pest insecticidal capacity, and an effective method for insecticide having an exceptional immediate effect within a short period of time on an agricultural pest due to filamentous fungi contained in a cell extract produced by the production method.

### Brief Description of Drawings

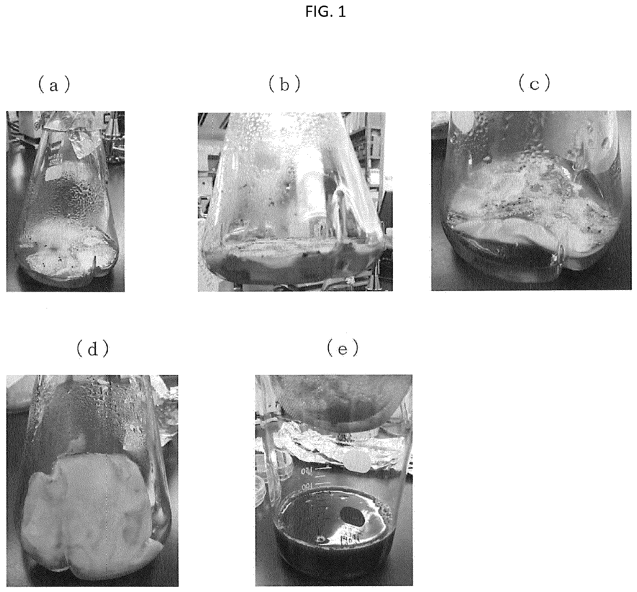

Fig. 1(a) is a photograph of a culture after liquid culture of HNT-01 strain. Fig. 1(b) is a photograph of a colony of HNT-01 strain. Fig. 1(c) is a photograph illustrating that the colony of HNT-01 strain is hydrophobic. Fig. 1(d) is a photograph of a membrane body of the colony of HNT-01 strain separated from the culture. Fig. 1(e) is a photograph of a spore solution of HNT-01 strain.

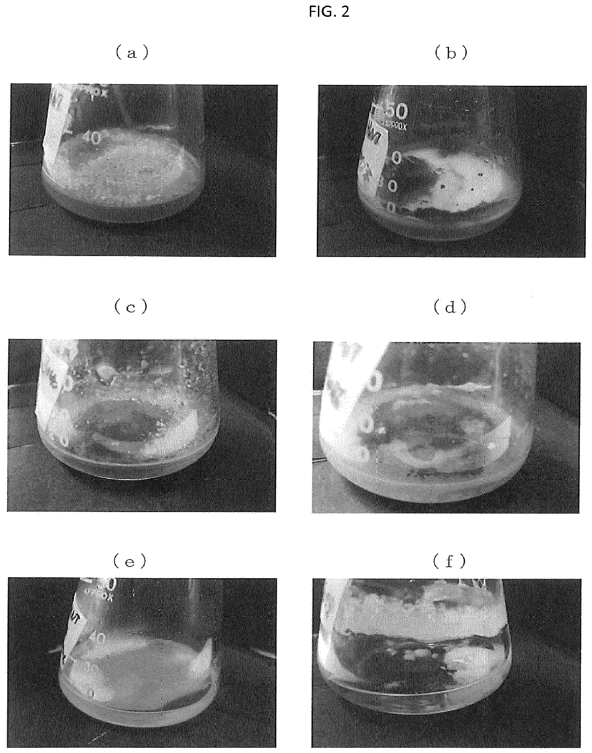

Figs. 2 (a) to 2 (f) are photographs illustrating growth of HNT-01 strain under different culture conditions. Fig. 2(a) is a photograph illustrating a result of culture in a PDB medium. Fig. 2(b) is a photograph illustrating a result of static culture in a PDB medium to which waste rice (grain of rice) has been added.
Fig. 2(c) is a photograph illustrating a result of static culture in a PDB medium to which chitin (powder) has been added. Fig. 2 (d) is a photograph illustrating a result of culture in a PDB medium to which Towada stone powder has been added. Fig. 2(e) is a photograph illustrating a result of culture in a PDB medium to which carboxymethyl cellulose has been added. Fig. 2 (f) is a photograph illustrating a result of shaking culture in a PDB medium.

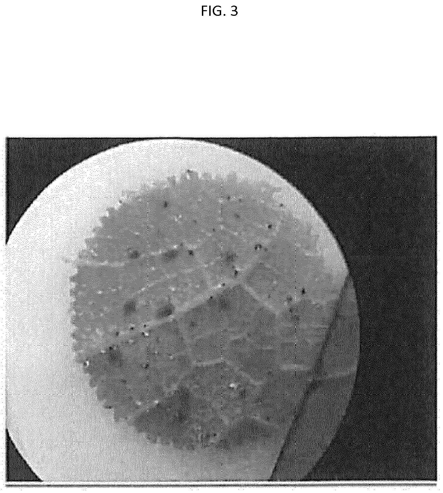

Fig. 3 is a photograph illustrating an influence of a spore solution of HNT-01 strain on Tetranychus urticae Koch.

### Description of Embodiments

The present invention relates to a method for producing a cell extract having agricultural pest insecticidal capacity and a method for insecticide of an agricultural pest due to filamentous fungi contained in a cell extract produced by the production method.

In the present embodiment, among filamentous fungi having agricultural pest insecticidal capacity, Trichoderma atroviride HNT-01 strain (hereinafter, referred to as "HNT-01 strain") was used.

HNT-01 strain is a fungus which was deposited to National Institute of Technology and Evaluation Patent Microorganisms Depositary (2-5-8, Kazusakamatari, Kisarazu, Chiba, Japan) dated on September 7, 2012, and to which the accession number NITE P-1419 was given.

HNT-01 strain grows fast and forms a yellow-green to green colony having a wool shape to a velvet shape. A conidiophore of HNT-01 strain branches regularly or irregularly, and the conidiophores are assembled to form a wool-like mass (branch) in a mature colony. In addition, HNT-01 strain has a morphological feature such as forming a green conidia mass at a tip of a phialide or forming a chlamydospore on a vegetative mycelium.

### <Recovery of HNT-01 strain spore solution>

HNT-01 strain was cultured by a method obtained by combining two different culture methods, and a spore solution thereof was recovered successfully.

Specifically, the spore solution of HNT-01 strain was recovered through the following steps.
- Step for culturing filamentous fungi in a solid medium and obtaining a solid culture of the filamentous fungi
- Step for culturing the filamentous fungi in a liquid medium and obtaining a liquid culture of the filamentous fungi
- Step for separating mycelia of the filamentous fungi from the liquid culture of the filamentous fungi
- Step for mixing the liquid culture from which the mycelia have been removed and the solid culture and obtaining a mixed culture
- Step for extracting a spore solution of the filamentous fungi from the mixed culture

### (1) Experimental material

- 10 g of waste rice
- 30 mL of PDB liquid medium
- HNT-01 strain culture agar medium
- sterile physiological saline

### (2) Experimental method

### (2)-1. Solid culture

10 g of waste rice was weighed in a conical beaker, 100 mL of tap water was poured thereinto, and the waste rice was allowed to absorb water overnight.

After water absorption, the tap water was discarded, 0.24 g of PDB was added, and an autoclave treatment was performed (121°C, 30 minutes). The next day, an autoclave treatment was performed again, and the waste rice was sterilized.

To the sterilized waste rice, 2 mL of sterile physiological saline was added, and the resulting mixture was stirred. A 1 cm-square HNT-01 strain culture agar fragment was inoculated thereinto. After inoculation, culture was performed at room temperature for 18 days.

### (2)-2. Liquid culture

A 30 mL PDB liquid medium was produced in a 300 mL flask, and a 1 cm-square HNT-01 strain culture agar fragment was inoculated thereinto. After inoculation, culture was performed at room temperature for 18 days.

### (3) Recovery of spore solution

A colony of HNT-01 strain was formed in the culture solution after liquid culture (Fig. 1(a)). As illustrated in Fig. 1(b), it was confirmed that the colony was formed not in the culture liquid but on a surface of the culture solution.

As illustrated in Figs. 1(c) and 1(d), it was confirmed that this colony formed a film-like thin membrane body completely separable from the culture solution.

This indicates that the film-like thin membrane body of HNT-01 strain is hydrophobic. This suggests that the number of spores increased due to increase in a secondary metabolite such as a surfactant in accordance with formation of a colony.

The film-like thin membrane body of HNT-01 strain was separated from the culture solution. The culture solution from which the membrane body had been removed was put into a culture after solid culture, and the resulting mixture was stirred with a platinum loop. After stirring, a spore solution of HNT-01 strain was filtered with a sterilized tea strainer and beaker to be recovered (Fig. 1(e)).

### <Search for high production medium of acaricidal substance>

In order to examine an agricultural pest insecticidal effect of HNT-01 strain, the number of spores of HNT-01 strain cultured with different media was measured.

### (1) Medium composition

medium 1: PDB medium
medium 2: PDB medium to which 0.6 g of waste rice (grain of rice) has been added
medium 3: PDB medium to which 0.6 g of chitin (powder) has been added
medium 4: PDB medium to which 0.3 g of Towada stone powder has been added
medium 5: PDB medium to which 0.1 g of carboxymethyl cellulose (CMC) has been added

### (2) Experimental method

By using a PDB medium mainly, a PDB addition medium to which each of waste rice (grain of rice), chitin (powder), Towada stone powder, and carboxymethyl cellulose (CMC) described above had been added was produced, and an agar fragment of HNT-01 strain was inoculated thereinto.

Towada stone is green tuff mined in Hinai, Odate, Akita (hereinafter, also referred to as "Towada stone"), and an official name thereof is "dacite pumice tuff" and is known under the name "green tuff".

Towada stone is a complex ore including quartz, albite, chlorite, or the like as a mineral composition, and contains a lot of minerals. Towada stone adsorbs and releases a substance because of porosity, for example, and is also used as an agricultural fertilizer. The main composition of Towada stone is illustrated in Table 1.

**[Table 1]**

| chemical composition of Towada stone (%) | | | | | | | |
|---|---|---|---|---|---|---|---|
| SiO₂ silicon oxide | Al₂O₅ aluminum oxide | Fe₂O₅ iron oxide | TiO₂ titanium oxide | CaO calcium oxide | MgO magnesium oxide | Na₂O sodium oxide | K₂O potassium oxide |
| 72.1 | 12.7 | 3.2 | 0.5 | 1.3 | 1.0 | 4.1 | 2.3 |

Culture was performed under the following conditions.
culture condition 1: static culture in medium 1 (room temperature, two weeks)
culture condition 2: static culture in medium 2 (room temperature, two weeks)
culture condition 3: static culture in medium 3 (room temperature, two weeks)
culture condition 4: static culture in medium 4 (room temperature, two weeks)
culture condition 5: static culture in medium 5 (room temperature, two weeks)
culture condition 6: shaking culture in medium 1 (room temperature, two weeks)

After culture, mycelia forming a film-like thin membrane body was separated from a culture solution by decantation.

To the mycelia, 10 mL of sterile physiological saline was added, and the resulting mixture was stirred to produce a spore solution. The spore solution was filtered in order to remove a solid, and the number of spores was measured.

### (3) Measurement result of the number of spores

Table 2 illustrates measurement results of the number of spores in each of the spore solutions obtained under the above culture conditions.

**[Table 2]**

| culture condition | medium | the number of spores (cells/ML) | order of growth progress by visual observation |
|---|---|---|---|
| 1 | 1 | 7.1×10⁷ | 4 |
| 2 | 2 | 3.5×10⁸ | 1 |
| 3 | 3 | 6.5×10⁷ | 3 |
| 4 | 4 | 5.7×10⁸ | 2 |
| 5 | 5 | 5.3×10⁵ | 6 |
| 6 | 1 | 7.0×10⁶ | 5 |

Figs. 2(a) to 2(f) illustrate growth of HNT-01 strain under culture conditions. As illustrated in Table 2, in the spore solution obtained under each of culture conditions 2 and 4, a dark green spore was observed after lapse of about one week.

In the spore solution obtained under culture condition 3, the color of the spore solution changed from yellow to dark green after lapse of about eight days.

From Table 2, it was confirmed that the number of spores of HNT-01 strain under each of culture conditions 2 and 4 was about 10 to 1000 times the number of spores of HNT-01 strain under each of the other four culture conditions.

HNT-01 strain under culture condition 1 and HNT-01 strain under culture condition 3 had almost the same number of spores, but were different in color of the spore solution (yellow-green and dark green) (Figs. 2(a) and 2(c)). It is considered that this largely reflects maturity rather than the number of spores.

### <Experimental Example 1> Application of spore solution of HNT-01 strain to agricultural pest

In this experimental example, an insecticidal effect of a spore solution of HNT-01 strain with respect to Tetranychus urticae Koch classified into Tetranychidae known as an agricultural pest was observed.

### (1) Biological material

- Tetranychus urticae Koch (red adult)
- HNT-01 strain
- Leaf of P. Vulgaris
- Leaf of Brassica oleracea var. capitata

### (2) Experimental Sample

sample 1: distilled water (control)
sample 2: spore solution A of HNT-01 strain
   a spore solution of HNT-01 strain cultured in a PDB medium to which Towada stone powder described above has been added (hereinafter, referred to as "HVT spore solution")
sample 3: spore solution B of HNT-01 strain
   a solution obtained by adding a Towada stone powder (particle diameter: 200 nm) aqueous solution having an equal amount to the spore solution to the spore solution of HNT-01 strain described in the above "Recovery of HNT-01 strain spore solution" (hereinafter, referred to as "HVT200 spore solution")
sample 4: a Towada stone powder (particle diameter: 200 nm) aqueous solution (hereinafter, referred to as "N200")

### (3) Experimental method

Three 2 cm-square leaves of P. Vulgaris were transferred to a large petri dish lined with cotton wool moistened with distilled water. Red adults of Tetranychus urticae Koch were transferred to the transferred leaves of P. Vulgaris such that the red adults were present at 20 adults/leaf. The red adults of Tetranychus urticae Koch were allowed to stand over night to be accustomed to the leaves of P. Vulgaris.

Thereafter, the above samples were subjected to an immersion test for five seconds. After the immersion test, the leaves of P. Vulgaris were dried for one hour, and observation was performed with a microscope. Thereafter, observation was performed every 24 hours.

### (4) Result

Table 3 illustrates a time-serial survival ratio of Tetranychus urticae Koch in each sample.

**[Table 3]**

| survival ratio of Tetranychus urticae Koch (%) | | | | |
|---|---|---|---|---|
| sample/observation time | after lapse of one hour | after lapse of 24 hours | after lapse of 48 hours | after lapse of 72 hours |
| Sample 1 | 100.0 | 82.6 | 75.0 | 60.5 |
| Sample 2 | 1.5 | 7.5 | 0.0 | 4.6 |
| Sample 3 | 0.0 | 11.9 | 8.1 | 4.8 |
| Sample 4 | 93.2 | 87.8 | 86.5 | 85.3 |

Use of the HVT spore solution or the HVT200 spore solution resulted in a numerical value as low as about 10% of a survival ratio of Tetranychus urticae Koch after lapse of 24 hours. After lapse of 72 hours, a survival ratio as low as about 5% was exhibited.

Table 4 illustrates the time-serial number of eggs laid by Tetranychus urticae Koch in each sample.

**[Table 4]**

| the number of eggs laid by Tetranychus urticae Koch (egg) | | | | |
|---|---|---|---|---|
| sample/observation time | after lapse of one hour | after lapse of 24 hours | after lapse of 48 hours | after lapse of 72 hours |
| Sample 1 | 0 | 49 | 137 | 253 |
| Sample 2 | 0 | 0 | 3 | 3 |
| Sample 3 | 0 | 0 | 4 | 4 |
| Sample 4 | 0 | 56 | 175 | 309 |

It was confirmed that use of the HVT spore solution or the HVT200 spore solution resulted in much more decrease in the number of eggs laid by Tetranychus urticae Koch depending on the survival ratio than other samples.

It is considered that this is because movement of Tetranychus urticae Koch is stopped by a metabolite derived from filamentous fungi having a higher concentration than in the natural world, and thereafter, the Tetranychus urticae Koch is infected with filamentous fungi, leading to death.

From this result, it was confirmed that
a spore solution of HNT-01 strain cultured in a PDB medium to which Towada stone powder described above has been added, or
a spore solution obtained by adding a Towada stone powder aqueous solution to a spore solution of HNT-01 strain obtained through
- a step for culturing filamentous fungi in a solid medium and obtaining a solid culture of the filamentous fungi,
- a step for culturing the filamentous fungi in a liquid medium and obtaining a liquid culture of the filamentous fungi,
- a step for separating mycelia of the filamentous fungi from the liquid culture of the filamentous fungi,
- a step for mixing the liquid culture from which the mycelia have been removed and the solid culture and obtaining a mixed culture, and
- a step for extracting a spore solution of the filamentous fungi from the mixed culture,
   was particularly suitable for insecticide of an agricultural pest.

### <Experimental Example 2> Application of supernatant of spore solution of HNT-01 strain to agricultural pest

In this experimental example, an insecticidal effect of a supernatant of a spore solution of HNT-01 strain with respect to Tetranychus urticae Koch was observed.

### (1) Biological material

- Tetranychus urticae Koch (red adult)
- HNT-01 strain

### (2) Experimental Sample

sample 1: supernatant A of a spore solution of HNT-01 strain A spore solution of HNT-01 strain cultured by a culture method described in the above "Recovery of HNT-01 strain spore solution"

sample 2: supernatant B of a spore solution of HNT-01 strain solution obtained by diluting the spore solution of sample 1 ten times
sample 3: supernatant C of a spore solution of HNT-01 strain solution obtained by diluting the spore solution of sample 1 100 times
sample 4: supernatant D of a spore solution of HNT-01 strain solution obtained by adding 1% olive oil to the spore solution of sample 1
sample 5: supernatant E of a spore solution of HNT-01 strain solution obtained by diluting the spore solution of sample 4 ten times
sample 6: supernatant F of a spore solution of HNT-01 strain solution obtained by diluting the spore solution of sample 4 100 times

### (3) Result

Table 5 illustrates a survival ratio of Tetranychus urticae Koch 60 minute after each sample was applied to Tetranychus urticae Koch. Three types were prepared for each sample.

**[Table 5]**

| | first observation | | second observation | | third observation | | the number of surviving Tetranychus urticaeKoch(the individual number) | the number of Tetranychus urticae Koch introduced(the individual number) | survival ratio (%) |
|---|---|---|---|---|---|---|---|---|---|
| | the number of surviving Tetranychus urticae Koch | the number of Tetranychus urticae Koch introduced | the number of surviving Tetranychus urticae Koch | the number of Tetranychus urticae Koch introduced | the number of surviving Tetranychus urticae Koch | the number of Tetranychus urticae Koch introduced | | | |
| Sample 1 | 0 | 8 | 0 | 10 | 0 | 10 | 0 | 28 | 0.0 |
| Sample 2 | 4 | 8 | 4 | 11 | 8 | 11 | 16 | 30 | 53.3 |
| Sample 3 | 5 | 10 | 6 | 8 | 7 | 10 | 18 | 28 | 64.3 |
| Sample 4 | 0 | 12 | 0 | 7 | 0 | 9 | 0 | 28 | 0.0 |
| Sample 5 | 0 | 10 | 0 | 9 | 0 | 12 | 0 | 31 | 0.0 |
| Sample 6 | 1 | 9 | 2 | 9 | 0 | 8 | 3 | 26 | 11.5 |

An insecticidal effect of Tetranychus urticae Koch was confirmed even with the supernatant of the spore solution of HNT-01 strain or a diluted solution thereof (samples 1 to 3).

An insecticidal effect of Tetranychus urticae Koch was confirmed even with the mixed solution obtained by adding olive oil to the supernatant of the spore solution of HNT-01 strain or a diluted solution thereof (samples 4 to 6).

Particularly, it was confirmed that addition of olive oil decreased survival of Tetranychus urticae Koch significantly.

It is considered that this is because a micelle-like substance is formed by a metabolite derived from filamentous fungi having a higher concentration than in the natural world and vegetable oil, this substance stops movement of Tetranychus urticae Koch, and thereafter, Tetranychus urticae Koch is infected with filamentous fungi, leading to death.

From this result, it was confirmed that
a supernatant of a spore solution of HNT-01 strain obtained through
- a step for culturing filamentous fungi in a solid medium and obtaining a solid culture of the filamentous fungi,
- a step for culturing the filamentous fungi in a liquid medium and obtaining a liquid culture of the filamentous fungi,
- a step for separating mycelia of the filamentous fungi from the liquid culture of the filamentous fungi,
- a step for mixing the liquid culture from which the mycelia have been removed and the solid culture and obtaining a mixed culture, and
- a step for extracting a spore solution of the filamentous fungi from the mixed culture,
   and a diluted solution thereof, or a mixed solution obtained by adding olive oil to the supernatant and a diluted solution thereof were particularly suitable for insecticide of an agricultural pest.

Hereinabove, preferable embodiments of the present invention have been described with reference to the drawings, but the present invention is not limited to the embodiments.

Fig. 3 illustrates an influence of a spore solution of HNT-01 strain on Tetranychus urticae Koch. Modification to various forms can be made in a technical scope understood from claims as long as a metabolite derived from filamentous fungi used for culture significantly limits an action of an agricultural pest by spiracle blockade, body surface abrasion, or both thereof, and thereafter Tetranychus urticae Koch is infected with the filamentous fungi used for the culture or indigenous filamentous fungi living in nature, leading to death.

## Claims

1. A method for producing a cell extract having agricultural pest insecticidal capacity, the method comprising:
(1) a step for culturing filamentous fungi in a solid medium and obtaining a solid culture of the filamentous fungi;
(2) a step for culturing the filamentous fungi in a liquid medium and obtaining a liquid culture of the filamentous fungi;
(3) a step for separating mycelia of the filamentous fungi from the liquid culture of the filamentous fungi;
(4) a step for mixing the liquid culture from which the mycelia have been removed and the solid culture and obtaining a mixed culture; and
(5) a step for extracting a spore solution of the filamentous fungi from the mixed culture.

2. The method for producing a cell extract according to claim 1, further comprising a step for mixing the spore solution and an inorganic mineral powder aqueous solution.

3. The method for producing a cell extract according to claim 1 or 2, wherein the filamentous fungi belong to the genus Trichoderma.

4. A method for insecticide of an agricultural pest, wherein a cell extract obtained by the production method according to any one of claims 1 to 3 is acted on an agricultural pest or a habitat thereof.

5. A method for insecticide of an agricultural pest, wherein a mixed solution obtained by adding vegetable oil to a supernatant of a cell extract obtained by the production method according to any one of claims 1 to 3 is acted on an agricultural pest or a habitat thereof.

6. A method for insecticide of an agricultural pest, wherein a mixed solution obtained by adding vegetable oil to a supernatant of a cell extract obtained by the production method according to any one of claims 1 to 3 is diluted, and the diluted mixed solution is acted on an agricultural pest or a habitat thereof.

7. The method for insecticide according to any one of claims 4 to 6, wherein the filamentous fungi belong to the genus Trichoderma.
